# EUROPÄISCHE PATENTSCHRIFT

(11) **EP 2 258 743 B1**
(45) Veröffentlichungstag und Bekanntmachung des Hinweises auf die Patenterteilung: **28.01.2015**
(21) Anmeldenummer: 10164678.4
(22) Anmeldetag: 02.06.2010
(51) Int. Cl.: C08G 63/82, C08K 3/10

(54) **Herstellung von Polyestern aus nachwachsenden Rohstoffen**
Production of polyesters from renewable resources
Fabrication de polyesters à partir de matières brutes renouvelables

(30) Priorität: 04.06.2009 EP 09161953
(43) Veröffentlichungstag der Anmeldung: 08.12.2010
(73) Patentinhaber: BASF SE, 67056 Ludwigshafen (DE)
(72) Erfinder: Steinke, Tobias Heinz, 67346, Speyer (DE); Görtz, Hans-Helmut, 67251, Freinsheim (DE); Mecking, Stefan, Dr. Prof., 78464 Konstanz (DE); Quinzler, Dorothee, 78462 Konstanz (DE)

(56) Entgegenhaltungen:
- DE-A1- 10 137 045
- GB-A- 2 226 822

## Beschreibung

Die vorliegende Erfindung betrifft ein Verfahren zur Herstellung von Oligo- oder Polyestern durch Reaktion von α,ω-Alkenolen mit Kohlenmonoxid in Anwesenheit mindestens eines Katalysators enthaltend Cobalt und mindestens einer Stickstoff enthaltenden Verbindung.

Üblicherweise werden Polyester durch Polykondensationsreaktion von Dialkoholen mit den entsprechenden Dicarbonsäureverbindungen hergestellt.

Die Ausgangsprodukte zur Herstellung der Polyester basieren dabei üblicherweise auf fossilen Materialien. Im Hinblick auf die begrenzte Menge der fossilen Materialien ist es wünschenswert, Polyester auf Basis alternativer, erneuerbarer Materialien herstellen zu können.

Ein viel versprechendes Ausgangsmaterial zur Herstellung von Polyestern stellt zum Beispiel Undecenol dar, das ausgehend von Rizinusöl (castor oil), einem Naturprodukt, das aus dem Samen des afrikanischen Wunderbaums, einem Wolfsmilchgewächs, gewonnen wird, hergestellt wird. Üblicherweise enthält Rizinusöl zu 80 bis 85 Gew.-% das Triglycerid der Rizinolsäure sowie weitere Glyceride verschiedener C₁₈-Fettsäuren. Aus dem Rizinusöl kann in zwei Schritten Undecenol gewonnen werden (F. C. Naughton, J. Am. Oil Chem. Soc., 1974, 51, 65 bis 71). Bei dem gewonnenen Undecenol handelt es sich um Undec-10-en-1-ol, also um ein α,ω-Alkenol.

Aus GB 2,226,822 A ist bekannt, α,ω-Alkenole mit Hilfe der Alkoxycarbonylierung, das heißt durch Umsetzung mit Kohlenmonoxid, in die entsprechenden Polyester umzuwandeln. Als Katalysatorsystem wird dabei ein System eingesetzt, das durch Kombination der folgenden Komponenten erhalten wird:
a) einer Palladium(II)-Verbindung,
b) einem monodentaten organischen Phosphin und/oder organischen Arsin und/oder organischen Stibin, gegebenenfalls in Kombination mit einem bidentaten Phosphin, Arsin oder Stibin und
c) einer protischen Säure, die einen pKa von weniger als 2 aufweist, wobei die folgenden Bedingungen erfüllt sein müssen:

Das molare Verhältnis der Komponente(n) (b) pro Gramm Atom Palladium ist ≥ 10, das molare Verhältnis der Komponente(n) (b) zu Komponente (c) ist > 1 und die Reaktionstemperatur, die angewendet wird ist < 140 °C. Gemäß den Beispielen in GB 2,226,822 weisen die erhaltenen Polyester verhältnismäßig geringe Molekulargewichte von 1700 bis 4000 auf.

Auch J. M. Penney, J. M. Boncella, Polymer Preprints, volume 39, no. 2, August 1998, American Chemical Society, Seite 573 betrifft die Herstellung von Polyestern durch Copolymerisation von ω-Undecenylalkohol und Kohlenmonoxid. Als Katalysator wird ein Katalysatorsystem aus ((C₆H₅)₃P)₂PdCl₂/SnCl₂ · 2H₂O eingesetzt. Gemäß der Beschreibung in J. M. Penney et al. werden Polyester mit Molekulargewichten im Bereich von 2000 bis 25000 erhalten, die bis auf 37000 bei Erhitzen unter Vakuum steigen können. Dabei ist jedoch nicht klar, ob sich diese Molekulargewichte auf die Reaktion von ω-Undecenylalkohol mit Kohlenmonoxid beziehen oder auf das alternative in J. M. Penney erwähnte Verfahren, worin Polyester durch Copolymerisation von Dekadien, einem Diol und Kohlenmonoxid erhalten werden.

Grundsätzlich sind zur Alkoxycarbonylierung neben Katalysatorsystemen, die Pd enthalten, andere Katalysatorsysteme geeignet. Beispielsweise betrifft WO 2008/023338 ein Verfahren zur Alkoxycarbonylierung durch Umsetzung von Olefinen mit Kohlenmonoxid und einem Alkohol in Anwesenheit eines Katalysators, der Cobalt enthält und in Anwesenheit eines Stickstoff enthaltenden Additivs. In dieser Reaktion werden jedoch keine Polyester, sondern einfache Ester erhalten.

Eine Schwierigkeit bei der Herstellung von Polyestern ausgehend von α,ω-Alkenolen ist die als Konkurrenzreaktion auftretende Laktonbildung anstelle der Bildung von Polyestern. Diese Problematik wird bereits in GB 2,226,822 A erwähnt, worin darauf hingewiesen wird, dass zur Herstellung von Polyestern der Einsatz eines speziellen katalytischen Pd-Systems sowie spezieller Reaktionsbedingungen erforderlich ist.

Aufgabe der vorliegenden Erfindung gegenüber dem vorstehend genannten Stand der Technik ist es, ein Verfahren zur Herstellung von Oligo- oder Polyestern bereitzustellen, ausgehend von α,ω-Alkenolen, insbesondere ausgehend von Undec-10-en-1-ol, das aus Rizinusöl gewonnen werden kann, wobei Polyester mit hohen Molekulargewichten erhalten werden können.

Die Aufgabe wird durch ein Verfahren zur Herstellung von Oligo- oder Polyestern durch Reaktion von α,ω-Alkenolen mit Kohlenmonoxid gelöst, wobei das Verfahren in Anwesenheit mindestens eines Katalysators enthaltend Cobalt und mindestens einer Stickstoff enthaltenden Verbindung durchgeführt wird.

Es wurde gefunden, dass mit Hilfe des vorstehend genannten Katalysators und der vorstehend genannten Stickstoff enthaltenden Verbindung die Herstellung von Oligo-oder Polyestern ausgehend von α,ω-Alkenolen möglich ist, wobei Polyester mit höheren Molekulargewichten als den in GB 2,226,822 A genannten Molekulargewichten erhältlich sind.

Ein weiterer Vorteil des erfindungsgemäßen Verfahrens ist, dass keine teuren Edelmetallverbindungen benötigt werden und keine teuren physiologisch bedenklich Phosphane eingesetzt werden, die zum Beispiel in dem Verfahren gemäß GB 2,226,822 A verwendet werden.

Unter α,ω-Alkenolen werden im Sinne der vorliegenden Anmeldung aliphatische Verbindungen verstanden, die eine Alkylenkette aufweisen, an deren einem Ende sich eine Doppelbindung befindet und deren anderem Ende sich eine OH-Gruppe befindet. Grundsätzlich ist die Kettenlänge der zwischen der Doppelbindung und der OH-Gruppe liegenden Alkylenkette beliebig und kann 2 bis 20 Kohlenstoffatome betragen. Bevorzugt weisen die α,ω-Alkenole die folgende allgemeine Formel (I) auf. 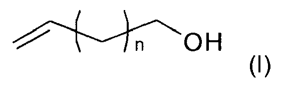 worin
n 1 bis 20, bevorzugt 2 bis 10, besonders bevorzugt 3 bis 8 bedeutet.

Besonders bevorzugt wird in den Verfahren gemäß der vorliegenden Erfindung als α ,ω-Alkenol Undec-10-en-1-ol eingesetzt, insbesondere Undec-10-en-1-ol, das aus Rizinusöl gewonnen wurde.

Unter Oligoestern werden Ester mit mindestens zwei Wiederholungseinheiten, bevorzugt 2 bis 10 Wiederholungseinheiten verstanden. Im Sinne der vorliegenden Erfindung werden die Ester mit > 10 Wiederholungseinheiten als Polyester bezeichnet.

Als Cobaltkatalysatoren sind in dem erfindungsgemäßen Verfahren alle dem Fachmann bekannten Cobaltkatalysatoren geeignet, insbesondere Cobaltkatalysatoren, die üblicherweise in Alkoxycarbonylierungsreaktionen eingesetzt werden. Bevorzugt handelt es sich bei dem Katalysator enthaltend Cobalt um einen Katalysator, der Cobaltcarbonyl enthält (Cobaltcarbonyl-Katalysator). Besonders bevorzugte Cobaltcarbonyl-Katalysatoren sind [Co₂(CO)₈], [Co₄(CO)₁₂] oder [HCo(CO)₄], besonders bevorzugt ist [Co₂(CO)₈].

Bei der in dem erfindungsgemäßen Verfahren eingesetzten mindestens einen Stickstoff enthaltenden Verbindung handelt es sich bevorzugt um eine Stickstoff enthaltende Verbindung mit einer heterocyclischen Struktur, worin mindestens ein Heteroatom der heterocyclischen Struktur Stickstoff ist. Die heterocyclische Struktur kann mindestens eine Doppelbindung zwischen zwei Ringatomen aufweisen. Bevorzugt ist die heterocyclische Struktur aromatisch. Ganz besonders bevorzugt handelt es sich bei der aromatischen heterocyclischen Struktur um Pyridin oder ein Pyridinderivat. Geeignete Pyridine oder Pyridinderivate umfassen Pyridin selbst, Alkyl-, Aryl- oder Amidosubstituierte Pyridine wie 2,4-, 3,4- 3,5-Lutidin und 4-Picolin, und Isochinoline. Besonders bevorzugt wird Pyridin als aromatische heterocyclische Struktur eingesetzt.

Das molare Verhältnis zwischen der Stickstoff enthaltenden heterocyclischen Verbindung, ganz besonders bevorzugt Pyridin, und Cobalt in dem erfindungsgemäß eingesetzten Katalysator beträgt im Allgemeinen 7 :1 bis 40 : 1, bevorzugt 8 : 1 bis 35 : 1.

Das molare Verhältnis des α,ω-Alkenol, ganz besonders bevorzugt Undec-10-en-1-ol, zu der molaren Menge Cobalt in dem erfindungsgemäß eingesetzten Katalysator enthaltend Cobalt beträgt im Allgemeinen 10 : 1 bis 300 : 1, bevorzugt 15 : 1 bis 250 : 1, besonders bevorzugt 20 : 1 bis 100 : 1, ganz besonders bevorzugt 30 : 1 bis 50 : 1.

In einer bevorzugten Ausführungsform wird das erfindungsgemäße Verfahren in Abwesenheit von weiterem Lösungsmittel - abgesehen von der Stickstoff enthaltenden Verbindung - durchgeführt. Überraschenderweise wurde gefunden, dass bei Anwesenheit von zum Beispiel Toluol als Lösungsmittel Oligo- oder Polyester mit wesentlich geringeren Molekulargewichten erhalten werden, als in Abwesenheit von weiterem Lösungsmittel.

Besonders gute Ergebnisse im Hinblick auf die Ausbeute und das Molekulargewicht werden in dem erfindungsgemäßen Verfahren dann erzielt, wenn das Verfahren bei einem Druck von > 50 bar durchgeführt wird. In einer bevorzugten Ausführungsform betrifft die vorliegende Erfindung daher das erfindungsgemäße Verfahren, wobei der Druck im Allgemeinen > 50 bar, bevorzugt > 50 bar bis 200 bar, besonders bevorzugt 150 bis 200 bar, beträgt. Auch die Temperatur des erfindungsgemäßen Verfahrens hat einen Einfluss auf die Ausbeute sowie das Molekulargewicht des erhaltenen Oligo-oder Polyesters. Im Allgemeinen wird das erfindungsgemäße Verfahren zur Erzielung guter Ausbeuten sowie hoher Molekulargewichte bei einer Temperatur von > 100 °C durchgeführt. Bevorzugt beträgt die Temperatur > 100 °C bis < 200 °C. Sowohl bei Temperaturen ab 200 °C als auch bei Temperaturen von 100 °C oder darunter werden Oligo- oder Polyester mit niedrigeren Ausbeuten und geringeren Molekulargewichten erhalten als in dem bevorzugt angegebenen Temperaturfenster. Ganz besonders bevorzugt wird das erfindungsgemäße Verfahren bei einem Druck von > 50 bar, bevorzugt > 50 bar bis 200 bar, besonders bevorzugt 150 bis 200 bar und bei einer Temperatur von > 100 °C, bevorzugt > 100 °C bis < 200 °C durchgeführt. In diesem Druck-/Temperaturbereich werden Polyester mit hohen Molekulargewichten in guten Ausbeuten erhalten.

Die mit Hilfe des erfindungsgemäßen Verfahrens erhaltenen Oligo- oder Polyester zeichnen sich durch vergleichsweise hohe Molekulargewichte aus und spezielle Verzweigungsgrade, so dass sich die Oligo- oder Polyester von mit Hilfe von anderen Katalysatorsystemen hergestellten Polyestern sowie von mit Hilfe anderer Herstellungsverfahren hergestellten Polyestern deutlich unterscheiden.

In dem erfindungsgemäß hergestellten Oligo- oder Polyester sind im Allgemeinen 5 bis 40 %, bevorzugt 10 bis 30 %, besonders bevorzugt 20 bis 30% der Wiederholungseinheiten des Oligo- oder Polyesters verzweigt.

Der erfindungsgemäß hergestellte Polyester weist im Allgemeinen ein zahlenmittleres Molekulargewicht Mₙ von ≥ 2000 g/mol, bevorzugt ≥ 3000 g/mol, besonders bevorzugt ≥ 7000 g/mol, ganz besonders bevorzugt ≥ 10000 g/mol auf. Das gewichtsmittlere Molekulargewicht Mw beträgt im Allgemeinen ≥ 3500 g/mol, bevorzugt ≥ 10000 g/mol, besonders bevorzugt ≥ 20000 g/mol, ganz besonders bevorzugt ≥ 25000 g/mol. Das zahlenmittlere und das gewichtsmittlere Molekulargewicht werden mittels GPC an Polystyrol Standard ermittelt. Der Verzweigungsgrad der erfindungsgemäß hergestellten Polyester wird mittels ¹H-NMR sowie ¹³C-NMR-Spektroskopie ermittelt.

Ein Vorteil der erfindungsgemäß hergestellten Oligo- oder Polyester ist zum einen, dass sie auf der Basis von erneuerbaren Materialien, zum Beispiel Undec-10-en-1-ol basierend auf Rizinusöl, hergestellt werden können. Ein weiterer Vorteil der erfindungsgemäß hergestellten Oligo- oder Polyester ist, dass die Oligo- oder Polyester grundsätzlich als bioabbaubare Oligo- oder Polymere geeignet sind. Dabei werden unter bioabbaubaren Polymeren im Sinne der vorliegenden Erfindung solche Materialien bezeichnet, die durch Mikroorganismen, Enzyme oder Hydrolyse, zum Beispiel im Boden abgebaut werden. Die Prüfung der biologischen Abbaubarkeit erfolgt durch die DIN-Norm EN 13432. Die bioabbaubaren Materialien müssen innerhalb von 6 bis 10 Wochen in einer Großkompostierung abgebaut werden. In einer weiteren Ausführungsform betrifft die vorliegende Erfindung somit einen erfindungsgemäß hergestellten Oligo- oder Polyester, der bioabbaubar ist.

Die erfindungsgemäß hergestellten Oligo- oder Polyester können grundsätzlich zur Herstellung von Folien, Fasern und Formkörpern verwendet werden.

Insbesondere können die erfindungsgemäß hergestellten Oligo- oder Polyester im Verpackungsbereich angewendet werden. Der Verpackungsbereich umfasst dabei zum Beispiel die Verpackung von Lebensmitteln, Getränken, Kosmetikartikeln sowie Medikamenten. Dabei werden die erfindungsgemäß hergestellten Oligo- oder Polyester insbesondere für Folienanwendungen eingesetzt. Die Produktion der entsprechenden Folien kann dabei gemäß dem Fachmann bekannten Verfahren erfolgen.

Die nachfolgenden Beispiele erläutern die Erfindung zusätzlich.

### Beispiele

### Beispiel 1

Ein 280 ml mechanisch gerührter Autoklav wird unter Inertgas mit den folgenden Materialien befüllt:

| | |
|---|---|
| Undecenol | 35 ml (175 mmol) |
| Co₂(CO)₈ | 820 mg (2,4 mmol) |
| Pyridin | 3 ml (38,4 mmol) |

Das Pyridin zu Cobalt Verhältnis beträgt 8 : 1.

Der Autoklav wird unter Druck mit Kohlenmonoxid versetzt, bis ein Druck von 200 bar erreicht wird, und auf eine Temperatur von 160 °C erhitzt. Die Reaktionsmischung wird für 69 Stunden bei dieser Temperatur gehalten. Anschließend wird auf Raumtemperatur abgekühlt. Nach Belüftung des Autoklaveninhalts wird ein rötlicher Feststoff erhalten, der zunächst mittels NMR analysiert wird durch Auflösen von etwa 20 mg des roten Feststoffs in deuteriertem Chloroform und Zugabe einer geringen Menge von Kohlenstoffdisulfid. Das Polymer (roter Feststoff) wird in Dichlormethan gelöst und aus Methanol ausgefällt, wobei 29,2 g des Polymers erhalten werden. Das Molekulargewicht (Mₙ = 1,0 x 10⁴ g/mol; M_{w} = 2,6 x 10⁴ g/mol) wird mittels Gelpermeationschromatographie (GPC) an einem Polymer Laboratories PL-GPC 50 Instrument mit zwei PL-Gel 5 µm MIXED-C Säulen in THF bei 40 °C gegen linearen Polystyrolstandard ermittelt. Die vorstehend erwähnte NMR Analyse ergibt ebenfalls ein Molekulargewicht Mₙ = von 1,0 x 10⁴ g/mol. Die thermische Analyse des Polymers wird mittels DSC (differential scanning calorimetry) unter Verwendung eines Netzsch DSC 204 F1 Phönix Instruments mit einer Heizrate von 10 K/min in einem Bereich von -100 °C bis 120 °C ermittelt. Es werden zwei Schmelzpunkte bei 57 °C und 64 °C in der zweiten Erhitzungskurve beobachtet.

### Beispiel 2

Ein 280 ml mechanisch gerührter Autoklav wird unter Inertgas mit den folgenden Materialien versetzt:

| | |
|---|---|
| Undecenol | 10 ml (50 mmol) |
| Co₂(CO)₈ | 233 mg (0,7 mmol) |
| Pyridin | 0,9 ml (11,2 mmol) |
| Toluol | 25 ml |

Das Pyridin zu Cobalt Verhältnis beträgt 8 : 1.

Die Reaktion wird wie in Beispiel 1 beschrieben durchgeführt mit der Ausnahme, dass die Reaktionszeit nur 64 Stunden beträgt. Die erhaltenen Produkte werden wie in Beispiel 1 analysiert. Nach Ausfällung werden 2,5 g Polymer mit einem Molekulargewicht Mₙ = 0,2 x 10⁴ und M_{w} = 0,4 x 10⁴ und einem Schmelzpunkt von Tₘ = 15 °C erhalten.

### Beispiel 3

Ein 15 ml magnetisch gerührter Autoklav wird unter Inertgasbedingungen mit den folgenden Materialien versetzt:

| | |
|---|---|
| Undecenol | 4 ml (20 mmol) |
| Co₂(CO)₈ | 94 mg (0,27 mmol) |
| Pyridin | 0,35 ml (4,39 mmol) |

Das Pyridin zu Cobalt Verhältnis beträgt 8 : 1.

Die Umsetzung wird wie in Beispiel 1 beschrieben durchgeführt, mit der Ausnahme, dass die Reaktionszeit nur 64 Stunden beträgt und der Kohlenmonoxiddruck 50 bar beträgt. Als Reaktionsprodukte werden Oligomere erhalten.

### Beispiel 4

Ein 280 ml mechanisch gerührter Autoklav wird unter Inertgasbedingungen mit den folgenden Materialien versetzt:

| | |
|---|---|
| Undecenol | 35 ml (175 mmol) |
| Co₂(CO)₈ | 820 mg (2,4 mmol) |
| Pyridin | 3 ml (38,4 mmol) |

Das Pyridin zu Cobalt Verhältnis beträgt 8 : 1.

Die Reaktion wird wie in Beispiel 1 beschrieben durchgeführt mit der Ausnahme, dass sie bei 200 °C durchgeführt wird und die Reaktionszeit nur 64 Stunden beträgt. Das erhaltene Polymer wird wie in Beispiel 1 beschrieben analysiert. Das ausgefällte Polymer hat ein Molekulargewicht von Mₙ = 0,3 x 10⁴ g/mol und M_{w} = 0,5 x 10⁴ g/mol und einen Schmelzpunkt von Tₘ = 14 °C.

### Beispiel 5

Ein 280 ml mechanisch gerührter Autoklav wird unter Inertgasbedingungen mit den folgenden Materialien versetzt:

| | |
|---|---|
| Undecenol | 35 ml (175 mmol) |
| Co₂(CO)₈ | 820 mg (2,4 mmol) |
| Pyridin | 12,4 ml (153,6 mmol) |

Das Pyridin zu Cobalt Verhältnis beträgt 32 : 1.

Der Autoklav wird unter Druck mit Kohlenmonoxid versetzt bis ein Druck von 180 bar erreicht wird und auf eine Temperatur von 160 °C geheizt. Diese Temperatur wird für 65 Stunden beibehalten. Anschließend wird auf Raumtemperatur gekühlt. Die Analyse des erhaltenen Polymers wird wie in Beispiel 1 beschrieben ausgeführt. Ausfällung von Methanol ergibt 28,3 g eines Polymers mit einem Molekulargewicht von Mₙ = 2,2 x 10⁴ g/mol und M_{w} = 4,7 x 10⁴ g/mol und zwei Schmelzpunkten von Tₘ = 61 °C und Tₘ = 66 °C.

### Beispiel 6

Ein 250 ml mechanisch gerührter Autoklav wird unter Inertgasbedingungen mit den folgenden Materialien versetzt:

| | |
|---|---|
| Undecenol | 35 ml (175 mmol) |
| Co₂(CO)₈ | 820 mg (2,4 mmol) |
| Pyridin | 12,4 ml (153,6 mmol) |

Das Pyridin zu Cobalt Verhältnis beträgt 32 : 1.

Der Autoklav wird unter Druck mit Kohlenmonoxid versetzt bis ein Druck von 50 bar erhalten wird und auf eine Temperatur von 100 °C erhitzt. Die Reaktionsmischung wird für 67 Stunden bei dieser Temperatur gehalten und anschließend auf Raumtemperatur gekühlt. Die Analyse des erhaltenen Polymers wird wie in Beispiel 1 beschrieben ausgeführt, abgesehen von der DSC Analyse. Die DSC Analyse erfolgt mit einer Heizrate von 30 K/min im Bereich von -50 °C bis 120 °C. Die Ausfällung aus Methanol ergab 5,8 g eines Polymers mit einem Molekulargewicht von Mₙ = 0,3 x 10⁴ g/mol und M_{w} = 0,3 x 10⁴ g/mol und einem Schmelzpunkt von Tₘ = 69 °C.

### Beispiel 7

Ein 280 ml mechanisch gerührter Autoklav wird unter Inertgasbedingungen mit den folgenden Materialien versetzt:

| | |
|---|---|
| Undecenol | 35 ml (175 mmol) |
| Co₂(CO)₈ | 820 mg (2,4 mmol) |
| Pyridin | 12,4 ml (153,6 mmol) |

Das Pyridin zu Cobalt Verhältnis beträgt 32 : 1.

Der Autoklav wird unter Druck mit Kohlenmonoxid versetzt bis ein Druck von 180 bar erhalten wird und auf eine Temperatur von 100 °C erhitzt. Die Reaktionsmischung wird für 67 Stunden bei dieser Temperatur gehalten und anschließend auf Raumtemperatur gekühlt. Bei diesem Versuch reagieren 19 % der Hydroxylgruppen unter Ausbildung von oligomeren Estern.

### Beispiel 8

Ein 280 ml mechanisch gerührter Autoklav wird unter Inertgasbedingungen mit den folgenden Materialien versetzt:

| | |
|---|---|
| Undecenol | 35 ml (175 mmol) |
| Co₂(CO)₈ | 820 mg (2,4 mmol) |
| Pyridin | 12,4 ml (153,6 mmol) |

Das Pyridin zu Cobalt Verhältnis beträgt 32 : 1.

Der Autoklav wird unter Druck mit Kohlenmonoxid versetzt bis ein Druck von 200 bar erhalten wird und auf eine Temperatur von 200 °C erhitzt. Die Reaktionsmischung wird für 66 Stunden bei dieser Temperatur gehalten und anschließend auf Raumtemperatur gekühlt. Die Analyse des erhaltenen Polymers erfolgt wie in Beispiel 1 beschrieben mit Ausnahme der thermischen Analyse, die wie in Beispiel 6 beschrieben erfolgt. Die Ausfällung aus Methanol ergibt 16,1 g eines Polymers mit einem Molekulargewicht von Mₙ = 0,6 x 10⁴ g/mol und M_{w} = 0,8 x 10⁴ g/mol und einem Schmelzpunkt von Tₘ = 61 °C.

### Beispiel 9

Ein 280 ml mechanisch gerührter Autoklav wird unter Inertgasbedingungen mit den folgenden Materialien versetzt:

| | |
|---|---|
| Undecenol | 35 ml (175 mmol) |
| Co₂(CO)₈ | 820 mg (2,4 mmol) |
| Pyridin | 12,4 ml (153,6 mmol) |

Das Pyridin zu Cobalt Verhältnis beträgt 32 : 1.

Der Autoklav wird unter Druck mit Kohlenmonoxid versetzt bis ein Druck von 100 bar erhalten wird und auf eine Temperatur von 160 °C erhitzt. Die Reaktionsmischung wird für 64 Stunden bei dieser Temperatur gehalten und anschließend auf Raumtemperatur gekühlt. Die Analyse des erhaltenen Polymers erfolgt wie in Beispiel 1 beschrieben mit Ausnahme der thermischen Analyse, die wie in Beispiel 6 beschrieben erfolgt. Die Ausfällung aus Methanol ergibt 27,2 g eines Polymers mit einem Molekulargewicht von Mₙ = 0,7 x 10⁴ g/mol und M_{w} = 1,1 x 10⁴ g/mol und einem Schmelzpunkt von Tₘ = 64 °C.

### Beispiel 10

Ein 280 ml mechanisch gerührter Autoklav wird unter Inertgas mit Undecenol, Co₂(CO)₈ und Pyridin versetzt:

| | |
|---|---|
| Verhältnis Undecenol zu Co₂(CO)₈: | 75 : 1 |
| Das Pyridin zu Cobalt Verhältnis beträgt | 32 : 1. |

Die Reaktion wird wie in Beispiel 1 beschrieben durchgeführt. Die erhaltenen Produkte werden wie in Beispiel 1 analysiert. Nach Ausfällung wird ein Polymer mit einem Molekulargewicht Mₙ = 15000 g/mol und M_{w} = 29000 g/mol und einem Schmelzpunkt von Tₘ bei 58°C und 66 °C erhalten.

### Beispiel 11

Ein 280 ml mechanisch gerührter Autoklav wird unter Inertgas mit Undecenol, Co₂(CO)₈ und Pyridin versetzt:

| | |
|---|---|
| Verhältnis Undecenol zu Co₂(CO)₈: | 18 : 1 |

Das Pyridin zu Cobalt Verhältnis beträgt 32 : 1.

Die Reaktion wird wie in Beispiel 1 beschrieben durchgeführt mit der Ausnahme, dass die Reaktionszeit 65 Stunden beträgt. Die erhaltenen Produkte werden wie in Beispiel 1 analysiert. Nach Ausfällung wird ein Polymer mit einem Molekulargewicht Mₙ = 13000 g/mol und M_{w} = 28000 g/mol und einem Schmelzpunkt von Tₘ von 61 °C erhalten.

### Beispiel 12

Ein 280 ml mechanisch gerührter Autoklav wird unter Inertgas mit Undecenol, Co₂(CO)₈ und Pyridin versetzt:

| | |
|---|---|
| Undecenol | 35 ml (175 mmol) |
| Co₂(CO)₈ | 120 mg (0,35 mmol) |
| Pyridin | 1,8 ml (22,4 mmol) |

Verhältnis Undecenol zu Co₂(CO)₈: 250 : 1

Das Pyridin zu Cobalt Verhältnis beträgt 32 : 1.

Die Reaktion wird wie in Beispiel 1 beschrieben durchgeführt mit der Ausnahme, dass die Reaktionszeit 66 Stunden beträgt. Die erhaltenen Produkte werden wie in Beispiel 1 analysiert. Nach Ausfällung wird ein Polymer mit einem Molekulargewicht Mₙ = 2400 g/mol und M_{w} = 3600 g/mol erhalten.

### Beispiel 13

Anstelle von Pyridin können auch verschiedene andere Stickstoffbasen als Promotoren verwendet werden.

Ein 280 ml mechanisch gerührter Autoklav wird unter Inertgas mit Undecenol, Co₂(CO)₈ und einer der nachstehend genannten Stickstoffbasen versetzt:

| | |
|---|---|
| Undecenol | 35 ml (175 mmol) |
| Co₂(CO)₈ | 820 mg (2,4 mmol) |

Das Stickstoffbase zu Cobalt Verhältnis beträgt 32 : 1.

Die Reaktionen werden wie in Beispiel 1 beschrieben bei einer Temperatur von 160°C durchgeführt mit der Ausnahme, dass die Reaktionen bei den in der nachstehenden Tabelle angegebenen Drucken (p) und Reaktionszeiten (t) durchgeführt werden und als Base anstelle von Pyridin die folgenden Stickstoffbasen eingesetzt werden:
i) 2,4-Lutidin
ii) 3,4-Lutidin
iii) 3,5-Lutidin
iv) 4-Picolin
v) Isochinolin
vi) Pyridin

Die erhaltenen Produkte werden wie in Beispiel 1 analysiert. Die Ergebnisse sind in der nachfolgenden Tabelle dargestellt:

| N° | Promoter | p [bar] | t [h] | M_{w} [GPC] | Mₙ [GPC] | Tₘ [°C] |
|---|---|---|---|---|---|---|
| i | 2,4-Lutidin 153,6 mmol | **190** | **22** | **3,1×10³** | **3,1×10³** | **60** |
| ii | 3,4-Lutidin 153,6 mmol | **160** | **20** | **5,5×10³** | **4,1×10³** | **46** / **63** |
| iii | 3,5-Lutidin 153,6 mmol | **180** | **17** | **11,2×10³** | **7,1×10³** | **64** |
| iv | 4-Picolin 153,6 mmol | **180** | **19** | **17,3×10³** | **9,7×10³** | **61** |
| v | Isoquinolin 153,6 mmol | **160** | **22** | **12,9×10³** | **7,6×10³** | **63** |
| vi | Pyridin 153,6 mmol | **160** | **18** | **23,1×10³** | **12,2×10³** | **63** |

## Patentansprüche

1. Verfahren zur Herstellung von Oligo- oder Polyestern durch Reaktion von α,ω-Alkenolen mit Kohlenmonoxid in Anwesenheit mindestens eines Katalysators enthaltend Cobalt und mindestens einer Stickstoff enthaltenden Verbindung.

2. Verfahren nach Anspruch 1, **dadurch gekennzeichnet, dass** die α,ω-Alkenole die folgende allgemeine Formel (I) aufweisen: 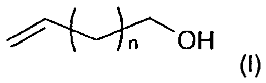 worin
n 1 bis 20, bevorzugt 2 bis 10, besonders bevorzugt 3 bis 8 bedeutet.

3. Verfahren nach Anspruch 1 oder 2, **dadurch gekennzeichnet, dass** das α,ω-Alkenole Undec-10-en-1-ol ist.

4. Verfahren nach einem der Ansprüche 1 bis 3, **dadurch gekennzeichnet, dass** der mindestens eine Katalysator enthaltend Cobalt ein Cobaltcarbonyl-Katalysator, bevorzugt [Co₂(CO)₈], [Co₄(CO)₁₂] oder [HCo(CO)₄], besonders bevorzugt [Co₂(CO)₈], ist.

5. Verfahren nach einem der Ansprüche 1 bis 4, **dadurch gekennzeichnet, dass** die mindestens eine Stickstoff enthaltende Verbindung eine heterocyclische Struktur aufweist, wobei mindestens ein Heteroatom der heterocyclischen Struktur Stickstoff ist, bevorzugt ist die heterocyclische Struktur aromatisch, besonders bevorzugt ist die aromatische heterocyclische Struktur Pyridin.

6. Verfahren nach einem der Ansprüche 1 bis 5, **dadurch gekennzeichnet, dass** das molare Verhältnis der Stickstoff enthaltenden Verbindung zu der Cobaltmenge des Katalysators 7 : 1 bis 40 : 1, bevorzugt 8 : 1 bis 35 : 1, beträgt.

7. Verfahren nach einem der Ansprüche 1 bis 6, **dadurch gekennzeichnet, dass** das Verfahren in Abwesenheit von weiterem Lösungsmittel - abgesehen von der Stickstoff enthaltenden Verbindung - erfolgt.

8. Verfahren nach einem der Ansprüche 1 bis 7, **dadurch gekennzeichnet, dass** das Verfahren bei einem Druck von > 50 bar, bevorzugt > 50 bar bis 200 bar, besonders bevorzugt 150 bar bis 200 bar, durchgeführt wird.

9. Verfahren nach einem der Ansprüche 1 bis 8, **dadurch gekennzeichnet, dass** das Verfahren bei einer Temperatur von > 100 °C, bevorzugt > 100 °C bis < 200 °C, durchgeführt wird.

## Claims

1. A process for preparing oligo- or polyesters by reacting α,ω-alkenols with carbon monoxide in the presence of at least one catalyst comprising cobalt and at least one compound comprising nitrogen.

2. The process according to claim 1 wherein the α,ω-alkenols have the following general formula (I) : 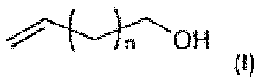 where
n is from 1 to 20, preferably from 2 to 10 and more preferably from 3 to 8.

3. The process according to either of claims 1 and 2 wherein the α,ω-alkenol is undec-10-en-1-ol.

4. The process according to any of claims 1 to 3 wherein the at least one catalyst comprising cobalt is a cobalt carbonyl catalyst, preferably [Co₂(CO)₈], [Co₄(CO)₁₂] or [HCo(CO)₄] and more preferably [Co₂(CO)₈].

5. The process according to any of claims 1 to 4 wherein the at least one compound comprising nitrogen has a heterocyclic structure wherein at least one heteroatom of the heterocyclic structure is nitrogen and the heterocyclic structure is preferably aromatic and the aromatic heterocyclic structure is more preferably pyridine.

6. The process according to any of claims 1 to 5 wherein the molar ratio of the compound comprising nitrogen to the amount of cobalt in the catalyst is from 7:1 to 40:1 and preferably from 8:1 to 35:1.

7. The process according to any of claims 1 to 6 wherein - apart from the compound comprising nitrogen - the process is effected in the absence of further solvent.

8. The process according to any of claims 1 to 7 wherein the process is carried out at a pressure of >50 bar, preferably from >50 bar to 200 bar and more preferably from 150 bar to 200 bar.

9. The process according to any of claims 1 to 8 wherein the process is carried out at a temperature of >100°C, preferably from >100°C to <200°C.

## Revendications

1. Procédé pour la préparation d'oligoesters ou de polyesters par réaction d'α,ω-alcénols avec du monoxyde de carbone en présence d'au moins un catalyseur contenant du cobalt et d'au moins un composé contenant de l'azote.

2. Procédé selon la revendication 1, **caractérisé en ce que** les α,ω-alcénols présentent la formule générale (I) suivante : 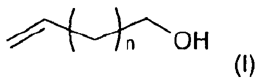 où
n vaut 1 à 20, de préférence 2 à 10, de manière particulièrement préférée 3 à 8.

3. Procédé selon la revendication 1 ou 2, **caractérisé en ce que** l'α,ω-alcénol est l'undéc-10-én-1-ol.

4. Procédé selon l'une quelconque des revendications 1 à 3, **caractérisé en ce que** ledit au moins un catalyseur contenant du cobalt est un catalyseur de type cobalt-carbonyle, de préférence [Co₂(CO)₈], [Co₄(CO)₁₂] ou [HCo(CO)₄], de manière particulièrement préférée [Co₂(CO)₈].

5. Procédé selon l'une quelconque des revendications 1 à 4, **caractérisé en ce que** ledit au moins un composé contenant de l'azote présente une structure hétérocyclique, au moins un hétéroatome de la structure hétérocyclique étant de l'azote, de préférence, la structure hétérocyclique est aromatique, de manière particulièrement préférée, la structure hétérocyclique aromatique est la pyridine.

6. Procédé selon l'une quelconque des revendications 1 à 5, **caractérisé en ce que** le rapport molaire du composé contenant de l'azote à la quantité de cobalt du catalyseur vaut 7:1 à 40:1, de préférence 8:1 à 35:1.

7. Procédé selon l'une quelconque des revendications 1 à 6, **caractérisé en ce que** le procédé a lieu en l'absence d'autres solvants - en dehors du composé contenant de l'azote.

8. Procédé selon l'une quelconque des revendications 1 à 7, **caractérisé en ce que** le procédé est réalisé à une pression > 50 bars, de préférence > 50 bars à 200 bars, de manière particulièrement préférée de 150 à 200 bars.

9. Procédé selon l'une quelconque des revendications 1 à 8, **caractérisé en ce que** le procédé est réalisé à une température > 100°C, de préférence > 100°C à < 200°C.
